## Europäisches Patentamt
## European Patent Office
## Office européen des brevets

(19)

(11) Numéro de publication: **0 092 697**
**B1**

(12)

# FASCICULE DE BREVET EUROPEEN

(45) Date de publication du fascicule du brevet:
**06.02.85**

(51) Int. Cl.⁴: **C 12 P 7/06, C 12 M 1/04**

(21) Numéro de dépôt: **83103283.4**

(22) Date de dépôt: **02.04.83**

(54) Procédé et fermenteur pour la production d'alcool.

(30) Priorité: **26.04.82 CH 2514/82**

(43) Date de publication de la demande:
**02.11.83 Bulletin 83/44**

(45) Mention de la délivrance du brevet:
**06.02.85 Bulletin 85/6**

(84) Etats contractants désignés:
**AT BE DE FR IT NL SE**

(56) Documents cités:
**FR - A - 788 126**
**FR - A - 2 082 617**
**GB - A - 552 428**
**GB - A - 1 417 487**
**GB - A - 2 066 843**

(73) Titulaire: **SOCIETE DES PRODUITS NESTLE S.A., Case postale 353, CH-1800 Vevey (CH)**

(72) Inventeur: **Kalina, Vladimir, Ch. du Devin 94, CH-1012 Lausanne (CH)**

ACTORUM AG

## Description

La présente invention a pour objet un procédé de production d'alcool par fermentation en continu d'un moût circulant en circuit fermé sous l'effet d'une pompe mammouth, du moût frais étant injecté en continu dans le circuit et du moût fermenté étant prélevé en continu du circuit.

La présente invention a également pour objet un fermenteur pour la production d'alcool en continu, comprenant une cuve de fermentation et une pompe mammouth branchées en circuit fermé, un dispositif d'injection de moût frais et un dispositif de prélèvement du moût fermenté.

On connaît plusieurs procédés et fermenteurs pour la production d'alcool en continu, dans lesquels la circulation d'un milieu de culture riche en sucre fermentescible que l'on désigne par le terme de moût dans le présent exposé est assurée à l'aide soit d'une pompe ordinaire, soit d'une pompe mammouth alimentée par l'intermédiaire d'un compresseur. On connaît même un procédé où la circulation est assurée par le seul dégagement du gaz carbonique produit au cours de la fermentation.

Dans ce dernier procédé connu, les conditions de fermentation varient fortement à mesure que l'on s'élève dans la cuve de fermentation, à cause de l'expansion et de la coalescence des bulles de gaz carbonique qui montent et, même si le rendement en poids d'alcool obtenu par rapport au poids de sucre fermentescible utilisé est bon, la productivité est faible comparée à la taille de l'installation.

Dans les autres procédés évoqués ci-dessus, on parvient à obtenir des productivités convenables aussi bien que de bons rendements proches du maximum théorique. Les taux de dilution, autrement dit le rapport entre la quantité de moût frais injecté par heure et la quantité de moût contenu dans le circuit, y sont respectables, à savoir d'une ou de plusieurs dizaines de pour-cent. Les teneurs en levure active du moût y sont de l'ordre de 50 g de matière sèche de levure par litre, le moût frais injecté y contient environ 100 à 150 g de sucres fermentescibles par litre, et le moût fermenté prélevé contient environ 6,5 à 8,5% d'alcool. Tous ces chiffres sont respectables et se retrouvent dans les meilleurs de ces procédés et fermenteurs connus.

Cependant, les dimensions de ces fermenteurs et les quantités de moût qui sont traités par ces procédés connus sont relativement modestes, à savoir de quelques litres à quelques mètres cubes. La raison doit en être cherchée dans le fait qu'il est très difficile de maintenir des conditions homogènes et favorables à la fermentation alcoolique dans un fermenteur de plusieurs dizaines, voire centaines de mètres cubes sans se heurter à de sérieux obstacles techniques et économiques. En effet, si l'on désire assurer un bon transfert à la levure du seul oxygène nécessaire à son métabolisme anaérobie, il est nécessaire d'assurer d'une part une bonne agitation du milieu et d'autre part une concentration adéquate de la phase gazeuse en oxygène. En augmentant les dimensions du fermenteur on augmente l'inhomogénéité des conditions régnant dans le milieu de fermentation, notamment en fonction de la hauteur de la cuve. Le rétablissement de cette homogénéité impliquerait pour les fermenteurs et les procédés connus des dispositifs compliqués d'injection de gaz de pression variable et/ou des dispositifs de brassage différentiels répartis régulièrement sur toute la hauteur de la cuve. Le maintien de cette homogénéité ne pourrait être assuré qu'en dépensant une très forte énergie de brassage, d'un coût prohibitif.

La présente invention a pour but de proposer un procédé et un fermenteur pour la production d'alcool en continu que l'on puisse réaliser à grande échelle sans rencontrer les obstacles techniques et économiques mentionnés ci-dessus.

A cet effet, le procédé selon la présente invention est caractérisé par le fait que l'on brasse le moût dans la partie inférieure d'une cuve de fermentation par injection d'un courant descendant de moût recyclé, on exerce une contrepression au sommet de la cuve afin que le gaz carbonique dégagé par la fermentation reste comprimé dans la partie supérieure de la cuve, on laisse le gaz carbonique exercer l'effet de pompage mammouth dans une colonne de pompage située au-dessus de la cuve, on recycle le moût en le laissant descendre en régime turbulent dans une conduite de retour et l'on injecte un mélange d'oxygène et de gaz inerte dans la partie supérieure de la conduite de retour.

De même, le fermenteur selon la présente invention est caractérisé par le fait que la cuve présente dans sa partie inférieure un dispositif de brassage hydraulique relié à une conduite de retour pour le moût recyclé, une vanne de contre-pression est prévue au sommet de la cuve pour empêcher l'expansion dans la cuve du gaz carbonique dégagé durant la fermentation, la pompe mammouth est disposée au-dessus de la vanne de contre-pression pour être actionnée par l'intermédiaire dudit gaz carbonique en expansion, la conduite de retour reliée au sommet de la pompe mammouth présente dans sa partie supérieure un dispositif d'injection de gaz pour l'alimentation du moût en oxygène, et cette conduite de retour est agencée de manière à assurer un écoulement turbulent du moût recyclé.

Les présents procédé et fermenteur permettent de réaliser l'objectif fixé et présentent en outre de multiples avantages. Leur conception est en particulier telle que la seule quantité d'oxygène nécessaire au travail anaérobie de la levure peut être injectée dans le moût. C'est ainsi que l'on peut injecter par heure une quantité dudit mélange gazeux contenant moins de 0,15 ml d'oxygène à pression amosphérique par gramme de poids sec de levure présente dans le moût. Ces quantités sont inférieures à celles qui sont préconisées dans les procédés connus. C'est en améliorant radicalement les conditions dans lesquelles on introduit l'oxygène dans le moût ainsi que les conditions régnant dans la cuve que l'on parvient ainsi à se passer de tout surplus d'oxygène et à éviter les risques d'enclenchement local de la fermentation anaérobie et les risques d'inhibition locale de la fermentation anaérobie.

Le moût peut donc contenir en solution dans la cuve de fermentation la quantité exacte d'oxygène nécessaire pour le travail anaérobie de la levure et l'agitation dans la cuve est suffisante pour que tout cet oxygène soit effectivement capté par la levure. On évite ainsi les phénomènes d'agglutination des levures autour des bulles de gaz dans la cuve de fermentation qui conduisent directement à une suralimentation en oxygène des cellules agglutinées. On évite également la dilution de l'oxygène dans les bulles de gaz qui s'élèveraient et se dilateraient en montant dans la cuve de fermentation, ce qui aurait pour conséquence contraire un affaiblissement du taux de transfert de l'oxygène dans le moût.

Le présent procédé et le présent fermenteur sont pour ainsi dire faits pour être réalisés à une grande échelle impliquant notamment une quantité de moût traité dans le circuit ou un volume d'au moins 50 m³. Plus la cuve est grande, plus la réalisation des conditions favorables est facilitée sans nécessiter la moindre dépense énergétique pour le brassage et la circulation.

Pour mettre en œuvre le présent procédé, on peut utiliser comme matière première un moût présentant de préférence une teneur en sucre fermentescible comprise entre 100 et 280 g/l outre les substances nutritives nécessaires à la levure. Cette dernière peut être choisie parmi les levures connues pour leurs bonnes aptitudes à la production d'alcool d'une part et à la formation d'agrégats qui facilitent leur sédimentation d'autre part.

On peut injecter du moût frais dans le bas du circuit, par exemple dans le bas de la conduite de retour pour bénéficier au maximum de l'effet de brassage de la cuve par le courant de moût recyclé. On injecte de préférence par heure dans le circuit une quantité de moût frais correspondant à 0,2-0,5 fois la quantité de moût présente dans le circuit, le moût dans le circuit pouvant contenir en moyenne 50 à 100 g de poids sec de levure par litre.

On a spécialement relevé plus haut que l'on peut injecter de l'oxygène dans le circuit en quantités très modérées couvrant uniquement le besoin réel en oxygène des levures pour leur travail anaérobie. Afin d'assurer une résorption complète de l'oxygène dans le moût, on peut veiller à ce que le temps de séjour du moût dans la conduite de retour soit suffisamment long et prévoir une turbulence du moût dans la partie supérieure de la conduite de retour suffisamment forte pour que le diamètre des bulles dudit mélange gazeux reste assez petit. C'est ainsi que, dans un mode de réalisation préféré du présent procédé, on prévoit d'une part que la turbulence du moût dans la partie supérieure de la conduite de retour soit suffisamment forte pour que le diamètre des bulles dudit mélange gazeux injecté dans le moût ne dépasse pas environ 4 mm au moment de leur formation et d'autre part que le temps de séjour du moût dans la conduite de retour soit d'au moins 10 s.

Le fait que l'on injecte un mélange gazeux plutôt que de l'oxygène pur par exemple a pour raison qu'il est possible en outre d'utiliser un gaz inerte qui se résorbe immédiatement dans le moût, laissant à l'état de bulle plus petite l'oxygène qu'il renfermait. Grâce au fait qu'on n'injecte pas plus d'oxygène que nécessaire, on peut obtenir que ces bulles résiduelles restent individuelles et se résorbent pour la plupart avant de se rencontrer et de former par coalescence des bulles plus grosses qui ne se résorberaient pas avant de pénétrer dans la cuve de fermentation.

On utilise donc le double effet de la turbulence du moût qui impose une taille limite aux bulles injectées et de la dilution de l'oxygène dans un gaz facilement résorbable qui permet de réduire rapidement le diamètre des bulles à une fraction de cette taille limite. Un mélange gazeux qui convient bien peut être réalisé avec l'air ambiant et le gaz carbonique dégagé durant la fermentation et comprend de préférence une partie en volume d'air et au moins cinq parties en volume de gaz carbonique.

Le moût recyclé est donc chargé d'oxygène pratiquement complètement résorbé et, optionnellement, il entraîne le moût frais lorsqu'il pénètre dans le bas de la cuve de fermentation avec une grande vitesse due à la hauteur de la conduite de retour. Cette énergie cinétique peut être transmise efficacement au moût se trouvant dans la cuve en concevant de manière adéquate la forme du bas de la cuve et l'embouchure de la conduite de retour.

Le moût peut s'élever lentement, à une vitesse d'environ 1-2 cm/s par exemple dans la cuve où l'on peut distinguer arbitrairement trois zones successives, à savoir une zone de brassage, une zone de sédimentation et une zone de concentration de $CO_2$. Dans la zone de brassage, le moût frais est donc intimement mélangé au moût recyclé et au moût présent dans le bas de la cuve. Dans la zone de sédimentation, qui représente la plus grande partie de la cuve, les levures travaillent efficacement sans être gênées ni par le brassage dans le fond de la cuve ni par le dégagement de $CO_2$. Les levures peuvent sédimenter plus vite que le moût ne monte et l'on peut donc observer un effet de concentration des levures dans le bas de la zone de sédimentation. Cela a pour conséquence que le moût recyclé contient moins de levure par litre que le moût dans la cuve de fermentation, ce qui facilite le fonctionnement d'un décanteur que l'on peut prévoir à l'extérieur de la cuve de fermentation, branché sur la conduite de retour par exemple, et ce qui diminue l'exposition des levures à l'oxygène durant leur trajet le long de la conduite de retour. Dans la zone de concentration de $CO_2$, comme dans toute la cuve de fermentation, le dégagement de $CO_2$ sous forme de bulles de gaz est empêché par la contre-pression exercée au sommet de la cuve, ce qui garantit des conditions de travail et de sédimentation homogènes et idéales pour les levures dans la zone de sédimentation.

Ce n'est donc qu'au-dessus de la cuve, dans une colonne de pompage, que l'on laisse le gaz carbonique se dégager du moût, former des bulles, se détendre et exercer l'effet de pompage mammouth. La vitesse de circulation dans cette colonne peut être très grande, de l'ordre d'au moins quelques mètres par seconde par exemple. A cette vitesse, la mousse formée est cassée et la séparation des gaz, autrement dit le dégazage, déjà réalisée en partie dans la colonne de pompage peut être achevée dans un dispositif efficace tel qu'un cyclone par exemple.

La conception du présent procédé permet également la réalisation d'un mode d'exécution particulier dans lequel on maintient au sommet du circuit une pression inférieure à la pression atmosphérique afin de prélever par évaporation l'alcool du moût fermenté. On peut bénéficier ainsi des avantages du système évaporatif, à savoir rendement plus élevé et moins d'inhibition due à l'alcool, sans devoir en supporter les inconvénients, à savoir la nécessité d'introduire de gros volumes d'air à pression réduite.

Une fois dégazé, le moût s'écoule en régime turbulent dans la conduite de retour. Pour assurer ce régime turbulent tout au long de la conduite de retour on doit pouvoir absorber certaines pertes de charge. C'est une des raisons pour lesquelles les présents procédé et fermenteur ont tout avantage à être réalisés à grande échelle, de manière à disposer des vitesses, des débits et des hauteurs nécessaires.

C'est sur cette conduite de retour que l'on soumet de préférence le moût recyclé au refroidissement nécessaire pour compenser l'échauffement provoqué par la fermentation dans la cuve. De même, c'est de cette conduite et notamment de sa partie inférieure que, dans un mode d'exécution sans système évaporatif, l'on prélève de préférence le moût fermenté chargé d'alcool. Le présent procédé permet là une décantation particulièrement efficace du fait que le moût a été dégazé au préalable et que la petite quantité de gaz carbonique qui lui a été injectée ensuite, le cas échéant, ne peut pas se désorber à cause de la pression exercée par la hauteur de la conduite de retour. On peut obtenir ainsi des vitesses de décantation de la levure de l'ordre de 20 cm/min. Une telle décantation permet d'une part de prélever du moût débarrassé de la levure et chargé d'alcool et d'autre part de sortir du circuit, le cas échéant, de la levure en excès. La levure décantée non en excès peut être recyclée dans la colonne de pompage par exemple sous le seul effet de la pression due à la hauteur de la conduite de retour, sans l'aide d'une pompe auxiliaire. Le moût décanté prélevé peut présenter une teneur en alcool d'environ 50 à 80 g/l, par exemple.

Quant au fermenteur selon la présente invention, il est donc caractérisé comme indiqué plus haut. En ce qui concerne ledit dispositif de brassage hydraulique prévu dans la partie inférieure de la cuve, il est de préférence du type Venturi. L'embouchure de la conduite de retour peut être à cet effet disposée au-dessus du fond de la cuve, dirigée vers le bas et entourée coaxialement d'une portion de tube de plus grand diamètre qu'elle même et évasé aux deux extrémités. Le fond de la cuve peut présenter lui-même une forme semi-sphérique, par exemple destinée à renforcer l'action du dispositif Venturi. La partie centrale de la cuve peut présenter alors une forme cylindrique dans le prolongement de la demi sphère du fond et être coiffée par une demi sphère complémentaire de celle du fond de manière à pouvoir canaliser le moût fermenté vers la vanne de contre-pression.

Cette dernière est de préférence à débit réglable de manière à pouvoir ajuster à volonté et contrôler le débit de la pompe mammouth et donc la vitesse de circulation du moût dans le circuit. Le diamètre et la hauteur de la pompe mammouth sont choisis en fonction du volume du fermenteur et du débit du moût à circuler. Ce débit est lui-même fonction de la chaleur dégagée par la réaction, il doit être assez grand pour que le gradient de température ne dépasse pas une certaine limite dans la cuve de fermentation. Cette limite est de l'ordre d'une différence d'environ 3° C entre le haut et le bas de la cuve.

De même, le dimensionnement de la conduite de retour est fait en fonction de la vitesse de circulation du moût et de manière à respecter de préférence les conditions de turbulence et de temps de séjour décrites plus haut. En ce qui concerne la turbulence, on peut l'influencer non seulement par le dimensionnement de la conduite, mais également en y prévoyant par exemple des diaphragmes ou des chicanes. Il faut simplement veiller à ce que la hauteur du fermenteur soit suffisante pour surmonter les pertes de charges y afférentes qui peuvent dépasser l'équivalent de 2 m de colonne d'eau.

Le présent fermenteur peut être muni à son sommet d'un dispositif de dégazage du type cyclone par exemple. En effet, la vitesse de circulation du moût dans sa partie supérieure est telle qu'elle permet l'usage de ce dispositif particulièrement efficace.

Le présent fermenteur présente de préférence un dispositif de refroidissement sur une partie, voire même sur la plus grande partie de sa conduite de retour. Ce dispositif peut être réalisé par exemple sous la forme d'un échangeur de chaleur tubulaire dans lequel le régime fortement turbulent de l'écoulement du moût se traduit par un coefficient de transfert de chaleur très élevé.

Le présent fermenteur peut être en outre avantageusement équipé d'un décanteur extérieur branché en amont sur le bas de la conduite de retour et en aval sur le milieu de la pompe mammouth par exemple. Le décanteur ainsi branché est capable de fonctionner très efficacement sans l'aide d'une pompe auxiliaire. Il est destiné d'une part à fournir du moût fermenté chargé en alcool mais débarrassé de ses levures et d'autre part à recycler la levure décantée tout en permettant d'évacuer, le cas échéant, la levure en excès.

C'est ainsi dans le bas de la conduite de retour que l'on branche de préférence une conduite d'amenée du moût frais, de manière que ce moût puisse être entraîné directement dans le dispositif de brassage par le moût recyclé.

Dans une forme d'exécution particulière du présent fermenteur, on prévoit de relier son sommet d'une part à un dispositif de pompage de gaz destiné à créer une pression inférieure à la pression atmosphérique et d'autre part à un dispositif de captage de vapeurs d'alcool. Dans cette forme d'exécution particulière, on peut prévoir également un dispositif de chauffage du sommet de la pompe mammouth afin de pouvoir mieux libérer les vapeurs d'alcool. Celles-ci seront mêlées au $CO_2$ dégagé du moût et devront en être séparées. C'est ainsi que le dispositif de captage des vapeurs d'alcool comprend de préférence une combinaison de moyens de condensation et de moyens de séparation de l'alcool et du $CO_2$.

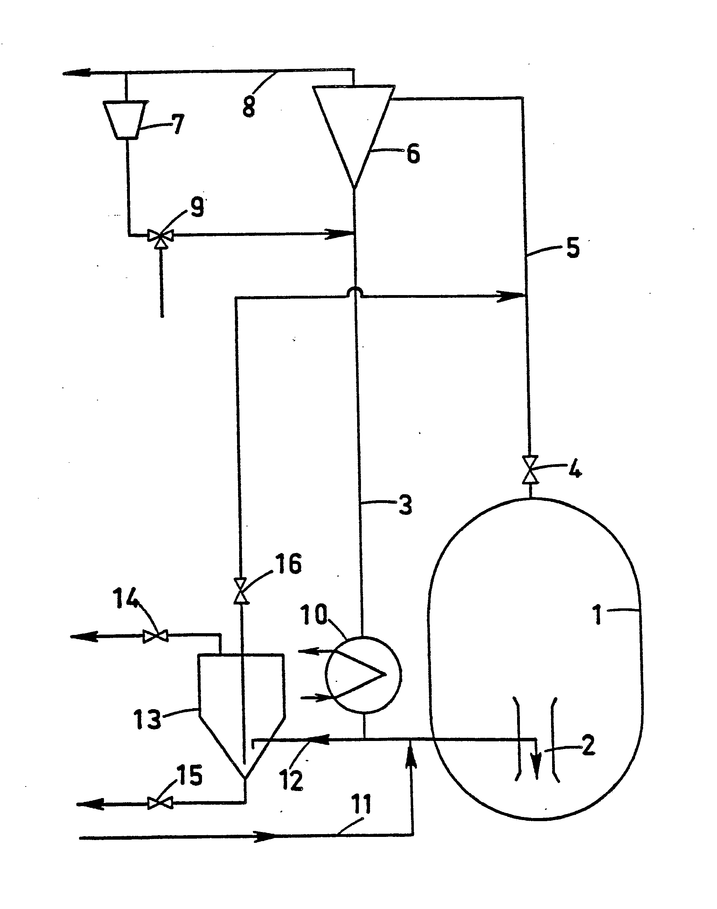

Le fermenteur selon la présente invention est décrit ci-après en référence au dessin annexé qui en représente schématiquement une forme d'exécution préférée.

Dans cette forme d'exécution, la cuve de fermentation 1 présente dans sa partie inférieure un dispositif de brassage 2 du type Venturi relié à la conduite de retour 3 pour le moût recyclé. La cuve présente à son sommet une vanne de contre-pression réglable 4 pour empêcher l'expansion dans la cuve du gaz carbonique dégagé durant la fermentation. La pompe mammouth 5 est disposée au-dessus de la vanne de contre-pression 4 pour être actionnée par l'intermédiaire de gaz carbonique délivré par ladite vanne. La conduite de retour 3 est reliée au sommet de la pompe mammouth 5 par l'intermédiaire d'un dispositif de dégazage 6 du type cyclone. Un petit compresseur 7 est branché sur la conduite d'évacuation 8 du $CO_2$ pour injecter dans le haut de la conduite de retour 3 un mélange d'air et de $CO_2$. L'air est introduit dans le dispositif d'injection de gaz par une vanne de mélange 9. La conduite de retour 3 passe par un échangeur de chaleur 10 pour le refroidissement du moût recyclé. Une conduite d'injection de moût frais 11 est branchée sur la conduite de retour 3 juste avant son entrée dans la cuve 1. De même, une conduite de prélèvement du moût chargé d'alcool et de levure 12 est branchée sur la conduite de retour au-dessous de l'échangeur de chaleur 10. Cette conduite 12 débouche dans un décanteur sous pression 13. Une vanne de prélèvement de moût fermenté décanté 14 permet d'ajuster la quantité de moût prélevé. Une vanne de vidange 15 est prévue pour écarter un excès éventuel de levure active. Enfin, une vanne de recyclage 16 permet de recycler dans la pompe mammouth 5, sous l'effet de la pression due à la hauteur de la conduite de recyclage 3, un moût chargé de levure récupérée dans le décanteur 13.

Le procédé de fermentation selon la présente invention est illustré ci-après par deux exemples dans lesquels la levure mise en œuvre est la levure *Saccharomyces cerevisiae* CBS 2961 dont les qualités de floculation peuvent être considérées comme relativement bonnes.

*Exemple 1 :*

On met en œuvre un mode préféré du procédé selon la présente invention dans un fermenteur correspondant au mode d'exécution représenté au dessin. Le fermenteur présente un volume total de 300 m³ et une hauteur totale de 23,5 m. La cuve de fermentation présente un fond et un sommet hémisphériques, une hauteur de 8,5 m et un diamètre d'environ 7,5 m. Les diamètres moyens respectifs de la pompe mammouth et de la conduite de retour sont d'environ 75 et 60 cm.

On injecte dans le bas de la conduite de retour 60 m³/h de moût frais contenant 150 g/l de sucre fermentescible. On injecte dans le haut de la conduite de retour 6 m³ d'air/h mélangés à 60 m³/h de $CO_2$ récupérés à la sortie du cyclone de dégazage. La teneur en levure active du moût dans le fermenteur est de 65 g en matière sèche par litre.

On règle le débit de la vanne de contre-pression sur la cuve de manière que la vitesse de circulation moyenne du moût soit d'environ 2 m/s dans la pompe mammouth, environ 6 m/s dans le cyclone de dégazage et environ 1,5 m/s dans la conduite de retour. Le temps de résidence moyen du moût dans la cuve est de 10 min. Le temps de séjour du moût dans la conduite de retour est en moyenne de 15 s. La turbulence dans la conduite de retour est telle que les bulles du mélange de gaz injecté présentent un diamètre qui ne dépasse pratiquement pas 3,5 mm dans le haut de la conduite.

On prélève du fermenteur 60 m³/h de moût présentant une teneur en alcool de 8,5% en volume.

*Exemple 2 :*

Dans un fermenteur analogue à celui représenté schématiquement au dessin mais comportant en outre à son sommet un dispositif de pompage des vapeurs d'alcool, on réalise un mode particulier du procédé selon la présente invention. Le fermenteur est rehaussé de 10 m par rapport à celui de l'exemple 1.

On injecte dans le bas de la conduite de retour 135 m³/h de moût frais contenant 265 g/l de sucre fermentescible. On injecte dans le haut de la conduite de retour 9 m³ d'air/h mélangés à 90 m³/h de $CO_2$ récupérés à la sortie du dispositif de pompage. La teneur en levure active du moût dans le fermenteur est de 50 g en matière sèche par litre.

On fait régner au sommet du fermenteur une pression de 70 mbar et l'on chauffe la partie supérieure de la pompe mammouth à une température de 36° C. On sépare les vapeurs d'alcool du $CO_2$ par un dispositif adéquat comprenant un piège à condensation et un séparateur à eau. On récupère 90% de l'alcool par ce moyen et les 10% restant par l'intermédiaire du décanteur. On obtient une productivité totale de 120 g d'alcool par litre de moût frais injecté.

## Revendications

1. Procédé de production d'alcool par fermentation en continu d'un moût circulant en circuit fermé sous l'effet d'une pompe mammouth, du moût frais étant injecté en continu dans le circuit et du moût fermenté étant prélevé en continu du circuit, caractérisé par le fait que l'on brasse le moût dans la partie inférieure d'une cuve de fermentation par injection d'un courant descendant de moût recyclé, on exerce une contre-pression au sommet de la cuve afin que le gaz carbonique dégagé par la fermentation reste comprimé dans la partie supérieure de la cuve, on laisse le gaz carbonique exercer l'effet de pompage mammouth dans une colonne de pompage située au-dessus de la cuve, on recycle le moût en le laissant descendre en régime turbulent dans une conduite de retour et l'on injecte un mélange d'oxygène et de gaz inerte dans la partie supérieure de la conduite de retour.

2. Procédé selon la revendication 1, caractérisé par le fait que l'on injecte par heure une quantité dudit mélange gazeux contenant moins de 0,15 ml d'oxygène à pression atmosphérique par gramme de poids sec de levure présente dans le moût.

3. Procédé selon la revendication 1, caractérisé par le fait que l'on obtient ledit mélange d'oxygène et de gaz inerte en mélangeant une partie en volume d'air et au moins cinq parties en volume de gaz carbonique.

4. Procédé selon la revendication 1, caractérisé par le fait que la turbulence du moût dans la partie supérieure de la conduite de retour est suffisamment forte pour que le diamètre des bulles dudit mélange gazeux injecté dans le moût ne dépasse pas environ 4 mm au moment de leur formation.

5. Procédé selon la revendication 1, caractérisé par le fait que le temps de séjour du moût dans la conduite de retour est d'au moins 10 s.

6. Procédé selon la revendication 1, caractérisé par le fait que la quantité de moût présente dans le circuit est d'au moins 50 m³.

7. Procédé selon la revendication 1, caractérisé par le fait que l'on injecte par heure dans le circuit une quantité de moût frais correspondant à 0,2 - 0,5 fois la quantité de moût présente dans le circuit, la teneur en sucre fermentescible du moût frais étant comprise entre 100 et 280 g/l.

8. Procédé selon la revendication 1, caractérisé par le fait que le moût dans le circuit contient en moyenne 50 - 100 g de poids sec de levure par litre.

9. Procédé selon la revendication 1, caractérisé par le fait que l'on maintient au sommet du circuit une pression inférieure à la pression atmosphérique afin de prélever par évaporation l'alcool du moût fermenté.

10. Fermenteur pour la production d'alcool en continu, comprenant une cuve de fermentation (1) et une pompe mammouth (5) branchées en circuit fermé, un dispositif d'injection de moût frais (11) et un dispositif de prélèvement du moût fermenté (13, 14), caractérisé par le fait que: la cuve (1) présente dans sa partie inférieure un dispositif de brassage hydraulique (2) relié à une conduite de retour (3) pour le moût recyclé, une vanne de contre-pression (4) est prévue au sommet de la cuve pour empêcher l'expansion dans la cuve de gaz carbonique dégagé durant la fermentation, la pompe mammouth (5) est disposée au-dessus de la vanne de contre-pression (4) pour être actionnée par l'intermédiaire dudit gaz carbonique en expansion, la conduite de retour (3) reliée au sommet de la pompe mammouth présente dans sa partie supérieure un dispositif d'injection de gaz (7, 8, 9) pour l'alimentation du moût en oxygène, et cette conduite de retour est agencée de manière à assurer un écoulement turbulent du moût recyclé.

11. Fermenteur selon la revendication 8, caractérisé par le fait que le dispositif de brassage (2) est du type Venturi.

12. Fermenteur selon la revendication 8, caractérisé par le fait qu'il présente un volume d'au moins 50 m³.

13. Fermenteur selon la revendication 8, caractérisé par le fait que son sommet est relié d'une part à un dispositif de pompage de gaz destiné à créer une pression inférieure à la pression atmosphérique et d'autre part à un dispositif de captage de vapeurs d'alcool.

## Claims

1. A process for the production of alcohol by the continuous fermentation of a must circulating in a closed circuit under the effect of a mammoth pump, fresh must being continuously injected into the circuit and fermented must being continuously removed from the circuit, wherein the must is stirred in the lower part of a fermentation vat by the injection of a descending flow of recycled must, a back pressure is applied to the top of the vat so that carbon dioxide gas which is released by the fermentation remains compressed in the upper part of the vat, the carbon dioxide gas is allowed to exert the mammoth pumping action in a pumping column located above the vat, the must is recycled by allowing it to descend in a turbulent flow in a return pipe, and a mixture of oxygen and inert gas is injected into the upper part of the return pipe.

2. A process according to claim 1, wherein a quantity of the said gaseous mixture containing less than 0.15 ml of oxygen under atmospheric pressure per gram of dry weight of yeast present in the must is injected per hour.

3. A process according to claim 1, wherein the said mixture of oxygen and inert gas is obtained by mixing one part by volume of air and at least five parts by volume of carbon dioxide gas.

4. A process according to claim 1, wherein the turbulence of the must in the upper part of the return pipe is strong enough for the diameter of the bubbles of the said gaseous mixture injected into the must not to exceed about 4 mm at the time of their formation.

5. A process according to claim 1, wherein the residence time of the must in the return pipe is at least 10 s.

6. A process according to claim 1, wherein the quantity of must which is present in the circuit is at least 50 m³.

7. A process according to claim 1, wherein a quantity of fresh must corresponding to from 0.2 to 0.5 times the quantity of must present in the circuit is injected per hour into the circuit, the content of fermentable sugar in the fresh must being from 100 to 280 g/l.

8. A process according to claim 1, wherein the must in the circuit contains on average from 50 to 100 g in dry weight of yeast per litre.

9. A process according to claim 1, wherein a pressure which is lower than atmospheric pressure is maintained at the top of the circuit in order to remove the alcohol from the fermented must by evaporation.

10. A fermenter for the continuous production of alcohol, comprising a fermentation vat (1) and a mammoth pump (5) which are connected in a closed circuit, a device for injecting fresh must (11) and a device for removing fermented must (13, 14), further comprising a hydraulic stirring device (2) in the lower part of the vat (1) connected to a return pipe (3) for the recycled must, and a back pressure valve (4) at the top of the vat to prevent expansion in the vat of carbon dioxide gas released during fermentation, the mammoth pump (5) being positioned above the back pressure valve (4) to be activated by means of the said carbon dioxide gas in expansion, the return pipe (3) connected to the top of the mammoth pump having in its upper part a gas injection device. (7, 8, 9) to supply the must with oxygen, and the said return pipe being disposed in such a manner as to ensure turbulent outflow of the recycled must.

11. A fermenter according to claim 10, wherein the stirring device (2) is of the Venturi type.

12. A fermenter according to claim 10, which has a volume of at least 50 m³.

13. A fermenter according to claim 10, wherein the top of the fermenter is connected on the one hand to a gas pumping device which is to produce a pressure lower than atmospheric pressure and on the other hand is connected to a device for collecting alcohol vapours.

**Patentansprüche**

1. Verfahren zur kontinuierlichen Herstellung von Alkohol durch Vergärung einer im geschlossenen Kreislauf unter der Einwirkung einer Mammutpumpe umlaufenden Maische, wobei frische Maische kontinuierlich in den Kreislauf eingeführt und vergorene Maische kontinuierlich aus dem Kreislauf abgezogen wird, dadurch gekennzeichnet, dass man die Maische im Unterteil eines Gärbehälters durch Einspritzen eines absteigenden Stromes der zurückgeführten Maische umrührt, dass man am Behälterkopf einen Gegendruck ausübt, so dass das durch die Gärung freigesetzte Kohlendioxidgas im Oberteil des Behälters komprimiert bleibt, dass man das Kohlendioxidgas in einer oberhalb des Behälters angeordneten Pumpkolonne einen Mammutpumpeneffekt ausüben lässt, dass man die Maische zurückführt, wobei man sie in einer Rückführleitung in turbulenter Strömung absteigen lässt, und dass man ein Gemisch aus Sauerstoff und Inertgas in den Oberteil der Rückführleitung einspritzt.

2. Verfahren nach Anspruch 1, dadurch gekennzeichnet, dass man je Stunde eine solche Menge des genannten Gasgemisches einspritzt, die weniger als 0,15 ml Sauerstoff bei Atmosphärendruck je Gramm Trockengewicht der in der Maische vorliegenden Hefe enthält.

3. Verfahren nach Anspruch 1, dadurch gekennzeichnet, dass man das genannte Gemisch aus Sauerstoff und Inertgas durch Vermischen eines Volumteiles Luft mit wenigstens 5 Volumteilen Kohlendioxidgas erhält.

4. Verfahren nach Anspruch 1, dadurch gekennzeichnet, dass die Turbulenz der Maische in dem oberen Teil der Rückführleitung ausreichend gross ist, so dass der Durchmesser der Blasen des genannten, in die Maische eingespritzten Gasgemisches etwa 4 mm zum Zeitpunkt ihrer Bildung nicht überschreitet.

5. Verfahren nach Anspruch 1, dadurch gekennzeichnet, dass die Verweilzeit der Maische in der Rückführleitung wenigstens 10 s beträgt.

6. Verfahren nach Anspruch 1, dadurch gekennzeichnet, dass die im Kreislauf befindliche Maischemenge wenigstens 50 m³ beträgt.

7. Verfahren nach Anspruch 1, dadurch gekennzeichnet, dass stündlich in den Kreislauf eine Menge an frischer Maische eingespritzt wird, die dem 0,2- bis 0,5fachen der im Kreislauf vorliegenden Maischemenge entspricht, wobei der Gehalt an fermentierbarem Zucker der frischen Maische zwischen 100 und 250 g/l liegt.

8. Verfahren nach Anspruch 1, dadurch gekennzeichnet, dass die Maische in dem Kreislauf im Mittel 50 bis 100 g Trockengewicht an Hefe je Liter enthält.

9. Verfahren nach Anspruch 1, dadurch gekennzeichnet, dass man im Kopfteil des Kreislaufes einen unter dem Atmosphärendruck liegenden Druck aufrecht erhält, um durch Verdampfen den Alkohol von der vergorenen Maische abzuziehen.

10. Fermenter zur kontinuierlichen Herstellung von Alkohol, umfassend einen Gärbehälter (1) und eine Mammutpumpe (5), die in einen geschlossenen Kreislauf eingebunden sind, eine Einspritzvorrichtung für frische Maische (11) und eine Entnahmevorrichtung für vergorene Maische (13, 14), dadurch gekennzeichnet, dass der Behälter (1) in seinem Unterteil eine hydraulische Rühreinrichtung (2) aufweist, die mit einer Rückleitung (3) für die zurückgeführte Maische verbunden ist, dass ein Gegendruckventil (4) am Behälterkopf angeordnet ist, um eine Expansion des während der Gärung freigesetzten Kohlendioxidgases im Behälter zur verhindern, dass die Mammutpumpe (5) oberhalb des Gegendruckventiles (4) angeordnet ist, um mittels dieses expandierenden Kohlendioxidgases betrieben zu werden, dass die mit dem Kopfteil der Mammutpumpe verbundene Rückleitung (3) in ihrem Oberteil eine Gaseinspritzeinrichtung (7, 8, 9) zur Versorgung der Maische mit Säuerstoff aufweist, und dass diese Rückleitung derart angeordnet ist, dass eine turbulente Strömung der rückgeführten Maische sichergestellt ist.

11. Fermenter nach Anspruch 10, dadurch gekennzeichnet, dass die Rühreinrichtung (2) vom Venturi-Typus ist.

12. Fermenter nach Anspruch 10, dadurch gekennzeichnet, dass er ein Volumen von wenigstens 50 m³ aufweist.

13. Fermenter nach Anspruch 10, dadurch gekennzeichnet, dass sein Kopfteil einerseits mit einer Gaspumpeinrichtung, die zur Ausbildung eines unter dem Atmosphärendruck liegenden Druckes bestimmt ist, und anderseits mit einer Auffangvorrichtung für Alkoholdämpfe verbunden ist.